(19) Europäisches Patentamt
European Patent Office
Office européen des brevets

(11) Publication number: **0 275 835 B1**

(12) **EUROPEAN PATENT SPECIFICATION**

(45) Date of publication of patent specification: **11.12.91**

(51) Int. Cl.$^5$: **C10M 135/36**, C07D 279/22

(21) Application number: **87810779.6**

(22) Date of filing: **23.12.87**

The file contains technical information submitted after the application was filed and not included in this specification

(54) **Substituted phenothiazines as lubricant stabilizers.**

(30) Priority: **31.12.86 US 948272**

(43) Date of publication of application:
**27.07.88 Bulletin 88/30**

(45) Publication of the grant of the patent:
**11.12.91 Bulletin 91/50**

(84) Designated Contracting States:
**BE DE ES FR GB IT NL**

(56) References cited:
**WO-A-88/02007**
**US-A- 3 376 224**
**US-A- 3 494 885**
**US-A- 3 536 706**

(73) Proprietor: **CIBA-GEIGY AG**
**Klybeckstrasse 141**
**CH-4002 Basel(CH)**

(72) Inventor: **Spivack, John D.**
**1 Blue Jay Street**
**Spring Valley New York 10977(US)**

EP 0 275 835 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to lubricant compositions which are stabilized against oxidative degradation by the presence therein of certain phenothiazine derivatives. It also relates to a number of novel phenothiazines within the broader group.

It is known to stabilize lubricants by the addition of antioxidants such, for example, as sterically hindered phenols, derivatives of p-phenylene diamine or of di-phenylamine in order to avoid decomposition, sludge formation, viscosity increases, and the like. US. Pat. No. 3,535,243 also discloses the use of diaminonaphthalenes as antioxidative additives for ester lubricants, while U.S. Pat. No. 3,536,706 discloses N-unsubstituted phenothiazines for a similar purpose.

It has now been discovered that certain derivatives of phenothiazine show surprisingly high stabilizer activity and sufficient solubility in a broad range of mineral and synthetic oils. Thus, the subject matter of the instant invention is a lubricant composition comprising mineral oils, synthetic oils, mixtures thereof, and the like, and as an antioxidative compound N-thioalkylenephenothiazines corresponding to the formula

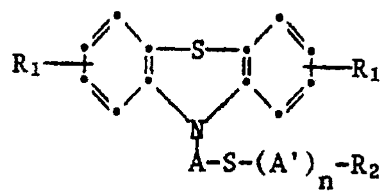

wherein $R_1$ are independently hydrogen, alkyl of 1 to 24 carbon atoms, cycloalkyl of 5 to 7 carbon atoms or aralkyl of 7 to 9 carbon atoms;
A and A′ are independently alkylene of 1 to 8 carbon atoms;
n is 0 or 1; and
$R_2$ is alkyl of 1 to 18 carbon atoms, cycloalkyl of 5 to 7 carbon atoms, said cycloalkyl substituted by alkyl of 1 to 18 carbon atoms, phenyl, phenyl substituted by alkyl, alkoxy or thioalkyl to a total carbon atom content of 7 to 30, naphthyl or naphthyl substituted by alkyl, alkoxy or thioalkyl to a total carbon atom content of 11 to 40, with the proviso that A is not ethylene when n is zero and $R_2$ is alkyl of 6 to 12 carbon atoms or phenyl.

Alkyl groups within the indicated definitions may be straight-chain or branched and may be methyl, ethyl, propyl, isopropyl, n-butyl, isobutyl, n-hexyl, n-octyl, 2-ethylhexyl, n-decyl, n-dodecyl, n-octadecyl or n-eicosyl. Cycloalkyl may be cylopentyl or cyclohexyl. Aralkyl may be benzyl, α-methylbenzyl, or α,α-dimethylbenzyl.

Alkylene groups include methylene, propylene, hexylene or octylene, but preferably correspond to the formula

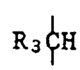

wherein $R_3$ is alkyl of 1 to 7 carbon atoms.

Preferred compounds include those wherein $R_1$ are independently hydrogen or alkyl of 1 to 12 carbon atoms; A and A′ are independently

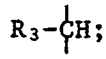

and $R_2$ is alkyl of 4 to 12 carbon atoms or phenyl or phenyl mono- or di- or trisubstituted by alkyl of 1 to 8 carbon atoms or with one of the substituents being alkoxy or thioalkyl of 1 to 12 carbon atoms.

Preferred compounds are N-(phenylthiomethylene)phenothiazine and N-(n-octylthiomethylene)-phenothiazine.

The N-thiomethylphenothiazine compounds may be prepared by reacting phenothiazine, formaldehyde and the appropriately substituted mercaptan or thiophenol in the presence of an alcoholic solvent such as methanol and ethanol at reaction temperatures ranging from 25 to 200 °C. The various starting materials

needed to prepare the indicated compounds are items of commerce or can be prepared by known methods.

The lubricant may be an oil or a grease based on mineral or synthetic oils, these lubricants being well known to those skilled in the art. The term mineral oil includes all mineral oils used for lubricant purposes, such as hydrocarbon mineral oils. The synthetic oil may be, for instance, an aliphatic or aromatic carboxylic ester, a polymeric ester, a polyalkylene oxide, a phosphoric acid ester, polyalphaolefins, or a silicone. Greases may be obtainable from these by adding metal soaps or similar thickeners.

The amount of phenothiazine compound added to the lubricant depends on the sensitivity of the oil base to oxidation and on the desired degree of protection. Generally, 0.01 to 2 % by weight will be added, and preferably 0.05 and 0.5 %. The compounds may be used in combination with other antioxidants known as oil additives.

The lubricant compositions of this invention may contain other additives which are incorporated to enhance the basic properties of lubricants still further. These further basic additives comprise: antioxidants, metal deactivators, rust inhibitors, viscosity index improvers, pour-point depressors, dispersants, surfactants and other extreme pressure additives and antiwear additives.

Examples of phenolic antioxidants

1. Alkylated monophenols

2,6-di-tert-butyl-4-methylphenol
2,6-di-tert-butylphenol
2-tert-butyl-4,6-dimethylphenol
2,6-di-tert-butyl-4-ethylphenol
2,6-di-tert-butyl-4-n-butylphenol
2,6-di-tert-butyl-4-sec-butylphenol
2,6-dicyclopentyl-4-methylphenol
2-(α-methylcyclohexyl)-4,6-dimethylphenol
2,6-di-octadecyl-4-methylphenol
2,4,6-tricyclohexylphenol
2,6-di-tert-butyl-4-methoxymethylphenol
o-tert-butylphenol

2. Alkylated hydroquinones

2,6-di-tert-butyl-4-methoxyphenol
2,5-di-tert-butyl-hydroquinone
2,5-di-tert-amyl-hydroquinone
2,6-diphenyl-4-octadecyloxyphenol

3. Hydroxylated thiodiphenyl ethers

2,2'-thio-bis(6-tert-butyl-4-methylphenol)
2,2'-thio-bis(4-octylphenol)
4,4'-thio-bis(6-tert-butyl-3-methylphenol)
4,4'-thio-bis(6-tert-butyl-2-methylphenol)

4. Alkylidene bisphenols

2,2'-methylene-bis(6-tert-butyl-4-methylphenol)
2,2'-methylene-bis(6-tert-butyl-4-ethylphenol)
2,2'-methylene-bis[4-methyl-6-(α-methylcyclohexyl)phenol]
2,2'-methylene-bis(4-methyl-6-cyclohexylphenol)
2,2'-methylene-bis(6-nonyl-4-methylphenol)
2,2'-methylene-bis(4,6-di-tert-butylphenol)
2,2'-ethylidene-bis(4,6-di-tert-butylphenol)
2,2'-ethylidene-bis(6-tert-butyl-4-isobutylphenol)
2,2'-methylene-bis[6-(α-methylbenzyl)-4-nonylphenol]

2,2'-methylene-bis[6-(α,α-dimethylbenzyl)-4-nonylphenol]
4,4'-methylene-bis-(2,6-di-tert-butylphenol)
4,4'-methylene-bis(6-tert-butyl-2-methylphenol)
1,1-bis-(5-tert-butyl-4-hydroxy-2-methylphenyl)-butane
2,6-di-(3-tert-butyl-5-methyl-2-hydroxybenzyl)-4-methylphenol
1,1,3-tris-(5'-tert-butyl-4'-hydroxy-2'-methylphenyl)-3-n-dodecylmercaptobutane
ethylene glycol bis[3,3-bis-(3'-tert-butyl-4'-hydroxyphenyl)-butyrate]
di-(3-tert-butyl-4-hydroxy-5-methylphenyl)-dicyclopentadiene
di-[2-(3'-tert-butyl-2'-hydroxy-5'-methylbenzyl)-6-tert-butyl-4-methylphenyl]terephthalate.

5. Benzyl compounds

1,3,5-tris(3,5-di-tert-butyl-4-hydroxybenzyl)-2,4,6-trimethylbenzene
bis(3,5-di-tert-butyl-4-hydroxybenzyl) sulfide
isooctyl 3,5-di-tert-butyl-4-hydroxybenzylmercaptoacetate
bis(4-tert-butyl-3-hydroxy-2,6-dimethylbenzyl) dithiolterephthalate
1,3,5-tris(3,5-di-tert-butyl-4-hydroxybenzyl) isocyanurate
1,3,5-tris(4-tert-butyl-3-hydroxy-2,6-dimethylbenzyl) isocyanurate
dioctadecyl 3,5-di-tert-butyl-4-hydroxybenzylphosphonate
calcium salt of monoethyl 3,5-di-tert-butyl-4-hydroxybenzylphosphonate.

6. Acylaminophenols

4-hydroxylauric anilide
4-hydroxystearic anilide
2,4-bisoctylmercapto-6-(3',5'-tert-butyl-4'-hydroxyanilino)-s-triazine
octyl N-(3,5-di-tert-butyl-4-hydroxyphenyl)carbamate

7. Esters of β-(3,5-di-tert-butyl-4-hydroxyphenyl)propionic acid

with monohydric or polyhydric alcohols, for example with methanol, octadecanol, 1,6-hexanediol, neopentyl glycol, thiodiethylene glycol,diethyleneglycol, triethylene glycol, pentaerythritol, trishydroxyethyl isocyanurate, dihydroxyethyloxalyldiamide.

8. Esters of β-(5-tert-butyl-4-hydroxy-3-methylphenyl)propionic acid

with monohydric or polyhydric alcohols, for example methanol, octadecanol, 1,6-hexanediol, neopentylglycol, thiodiethyleneglycol, diethylene glycol, triethyleneglycol, pentaerytritol, trishydroxyethyl isocyanurate or dihydroxyethyl oxalyldiamide.

9. Amides of β-(3,5-di-tert-butyl-4-hydroxyphenyl)propionic acid,

for example
N,N'-bis(3,5-di-tert-butyl-4-hydroxyphenylpropionyl)hexamethylenediamine
N,N'-bis(3,5-di-tert-butyl-4-hydroxyphenylpropionyl)trimethylenediamine
N,N'-bis(3,5-di-tert-butyl-4-hydroxyphenylpropionyl)hydrazine.

Examples of amine antioxidants:

N,N'-diisopropyl-p-phenylenediamine
N,N'-di-sec-butyl-p-phenylenediamine
N,N'-bis(1,4-dimethylpentyl)-p-phenylenediamine
N,N'-bis(1-ethyl-3-methylpentyl)-p-phenylenediamine
N,N'-bis(1-methylheptyl)-p-phenylenediamine
N,N'-diphenyl-p-phenylenediamine
N,N'-di(naphthyl-2-)-p-phenylenediamine
N-isopropyl-N'-phenyl-p-phenylenediamine
N-(1,3-dimethylbutyl)-N'-phenyl-p-phenylenediamine

N-(1-methylheptyl)-N'-phenyl-p-phenylenediamine
N-cyclohexyl-N'-phenyl-p-phenylenediamine
bis-4-(toluenesulfonamidophenyl)amine
N,N'-dimethyl-N,N'di-sec-butyl-p-phenylenediamine
diphenylamine
N-allyldiphenylamino
phenyl-α(β)-naphthylamine
4-isopropoxydiphenylamine
N-phenyl-1-naphthylamine
N-phenyl-2-naphthylamine
octylated diphenylamine
4-n-butylaminophenol
4-n-butyrylaminophenol
4-nonanoylaminophenol
4-dodecanoylaminophenol
4-octadecanoylaminophenol
di-(4-methoxyphenyl)amine
2,6-di-tert-butyl-4-dimethylaminomethylphenol
2,4-diaminodiphenylmethane
4,4'-diaminodiphenylmethane
N,N,N'N'-tetramethyl-4,4'-diaminodiphenylmethane
1,2-di(phenylamino)ethane
1,2-di-[(2-methylphenyl)amino]ethane
(o-tolyl)biguanide
di-[4-(1',3'-dimethylbutyl)phenyl]amine
tert-octylated N-phenyl-1-naphthylamine
mixture of mono- and dialkylated tert-butyl- and tert-octyldiphenylamines
p-tert.-octylphenyl-α-naphtylamine
p,p'-di-tert.octyldiphenylamine.

Others

2,3-dihydro-3,3-dimethyl-4H-1,4-benzothiazine
phenothiazine
N-allylphenothiazine
hindered phenols such as neopentanetetrayl tetrakis(3,5-di-tert-butyl-4-hydroxyhydrocinnamate)
n-octadecyl-3,5-di-tert-butyl-4-hydroxyhydrocinnamate
2,6-di-tert-butyl-p-cresol
2,2'-ethylidene-bis (4,6-di-tert-butylphenol)
aliphatic or aromatic phosphites; esters of thiodipropionic or thiodiacetic acid; or salts of dithiocarbamic or dithiophosphoric acids.

Such antioxidant combinations may show a synergistic action, i.e., the stabilizing effect of such a mixture being greater than the sum of the performance of the individual antioxidants. Such synergistic performances can be obtained when combining the instant compounds with certain aromatic amines or hindered phenols or with both types of antioxidants.

The lubricant composition may also contain other additives.

Examples of metal deactivators are:

for copper, e.g.: triazole, benzotriazole and derivatives thereof, 2-mercaptobenzotriazole, 2,5-dimercaptothiadiazole, 5,5'-methylenebisbenzotriazole, 4,5,6,7-tetrahydrobenzotriazole, salicylidene propylenediamine, salts of salicylaminoguanidine.

Examples of rust inhibitors are:

a) Organic acids, the esters, metal salts and anhydrides thereof, e.g.: N-oleylsarcosine, sorbitan monooleate, lead naphthenate, dodecenylsuccinic anhydride, monoalkenyl succinate, 4-nonylphenoxyacetic acid.

b) Nitrogen-containing compounds, for example:

I. Primary, secondary or tertiary aliphatic or cycloaliphatic amines and amine salts of organic and inorganic acids, for example oil-soluble alkylammonium carboxylates.

II. Heterocyclic compounds, e.g. substituted imidazolines and oxazolines.

c) Phosphorous-containing compounds, for example: amine salts of phosphoric acid partial esters.

d) Sulfur-containing compounds, for example: barium dinonylnaphthalenesulfonates, calcium petroleum sulfonates.

Examples of viscosity index improvers are:

polyacrylates, polymethylacrylates, polyvinylpyrrolidones, vinyl pyrrolidone/methacrylate copolymers, polybutenes, olefin copolymers, styrene/acrylate copolymers, polyethers.

Examples of pour-point depressors are:

polymethacrylates, alkylated naphthalene derivatives.

Examples of dispersants/surfactants are:

polybutenylsuccinimides, polybutenylphosphonic acid derivatives, basic magnesium, calcium and barium sulfonates and phenolates.

Examples of anti-wear additives are:

compounds which contain sulfur and/or phosphorous and/or halogen, such as sulfurised vegetable oils, zinc dialkyldithiophosphates, tritolylphosphate, chlorinated paraffins, alkyl disulfides, aryl disulfides, triphenylphosphorothionates, diethanolaminomethyltolyltriazole and di(2-ethylhexyl)aminomethyl tolyltriazole.

Said additives being widely known and used in lubricants.

The present invention comprises novel phenothiazine compounds with the formula

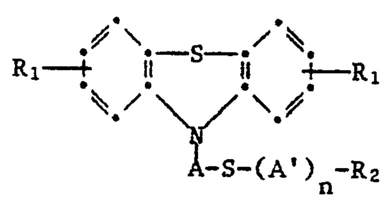

wherein $R_1$ are independently hydrogen, alkyl of 1 to 24 carbon atoms, cycloalkyl of 5 to 7 carbon atoms or aralkyl of 7 to 9 carbon atoms;
A and A′ are independently alkylene of 1 to 8 carbon atoms;
n is 0 or 1; and
$R_2$ is cycloalkyl of 5 to 7 carbon atoms, said cycloalkyl substituted by alkyl of 1 to 18 carbon atoms, phenyl, phenyl substituted by alkyl, alkoxy or thioalkyl to a total carbon atom content of 7 to 30, naphthyl or naphthyl substituted by alkyl, alkoxy or thioalkyl to a total carbon atom content of 11 to 40.

Preferred compounds include those wherein $R_1$ are independently hydrogen or alkyl of 1 to 12 carbon atoms;
A and A′ are independently

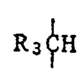

wherein $R_3$ is alkyl of 1 to 7 carbon atoms; and
$R_2$ is phenyl or phenyl mono-, di- or trisubstituted by alkyl of 1 to 8 carbon atoms or one of said substituents being alkoxy or thioalkyl of 1 to 12 carbon atoms.

Preferred compounds are N-(phenylthiomethylene)phenothiazine,
N-(benzylthiomethylene)phenothiazine,

N-(4-tert-butylphenylthiomethylene)phenothiazine,
N-(2,4-dimethylphenylthiomethylene)phenothiazine,
N-(4-methoxyphenylthiomethylene)phenothiazine and
N-(2-naphthylthiomethylene)phenothiazine.

Phenothiazine compounds within the generic formula wherein $R_2$ is alkyl have been previously disclosed. For example, U.S. Pat. No. 3,494,885 discloses N-substituted phenothiazines wherein the N-substitution is $C_2$-$C_{18}$alkylenethioalkyl for use as antioxidants in polycarbonates and polyesters. Similarly N-alkylenethioalkylphenothiazines have been disclosed in Winthrop et al, J. Am. Chem. Soc. 80, 4331-33 (1958) for a pharmacological utility.

Accordingly, the novel phenothiazines forming part for the instant invention correspond to the above noted generic formula with the exception that the $R_2$ definition does not include alkyl. The preferred substituents are also as defined above, excepting $R_2$ as alkyl, and the methods of preparation are, of course, as described hereinabove.

Compounds of this invention are also effective in stabilizing other organic materials such as plastics, polymers and resins in addition to the mineral and synthetic fluids.

Substrates in which the compounds of this invention are particularly useful are polyolefins such as polyethylene and polypropylene, polystyrene, including impact polystyrene, ABS resin, SBR, isoprene, as well as natural rubber, polyesters including polyethylene terephthalate and polybutylene terephthalate, including copolymers.

These stabilized polymer composition may optionally also contain various conventional additives such as antioxidants including alkylated monophenols, alkylated hydroquinones, hydroxylated thiodiphenyl ethers, alkylidene-bisphenols, various benzyl compounds, acylaminophenols and esters and amides of hindered hydroxyphenyl propionic acids; UV absorbers and light stabilizers including hydroxyphenyl benzotriazoles, benzophenones, benzoic acid esters, acrylates, nickel compounds, sterically hindered amines and oxalic acid diamides.

The following examples will further illustrate the embodiments of the instant invention.

Example I: N-(Phenylthiomethylene)phenothiazine

Phenothiazine (19.9 grams, 0.100 mol) is dispersed in 150 ml of ethanol and benzenethiol (11.4 grams, 0.101 moles) is added to the dispersion followed by 37.4 % formaldehyde (8.9 grams, 0.111 moles).

The dispersion is heated for 24 hours, further heating indicating that no additional reaction is taking place. The solvent is removed by distillation at 20 mm Hg pressure and unreacted benzenethiol removed at reduced pressure. The residue (28 grams) is crystallized successively from n-heptane, and a mixture of ethanol-cyclohexane yielding a total of 14.6 grams (45 % yield) of the desired product as white crystals.
Anal. Calcd. for $C_{19}H_{15}NS_2$: C, 70.99; H, 4.70; N, 4.36
Found: C, 70.8; H, 4.7; N, 4.3

Example II: N-(n-Octylthiomethylene)phenothiazine

Formaldehyde (37 wt. %, 6.9 grams, 0.111 moles) is added to a dispersion of phenothiazine (19.9 grams, 0.100 moles) in 250 ml of methanol. n-Octyl mercaptan (15.0 grams, 0.100 moles) is then added and the mixture stirred at reflux for about 30 hours. The solvent is removed at reduced pressure and the resulting residue dissolved in 400 ml of toluene and washed with water and dried over sodium sulfate. The dried toluene solution is freed of solvent under pressure with the residue being dissolved in 150 ml of refluxing n-heptane and allowed to cool to room temperature. Thin layer chromatography shows the crystalline precipitate (6.8 grams) to be phenothiazine. Removal of the heptane by distillation of the n-heptane under vacuum yields 25.6 grams of a brown residual liquid. This residual liquid is purified by High Presure Liquid Chromatography, yielding 18.2 grams of yellow liquid (51 % yield).
Anal. Calcd. for $C_{21}H_{27}NS_2$: C, 70.54; H, 7.61; N, 3.92; S, 17.93
Found: C, 70.4; H, 7.9; N, 4.1; S, 17.8

Example III: N-(Benzylthiomethylene)phenothiazine

The compound of Example III is prepared by following the general procedure of Example I and reacting benzylthiol with formaldehyde and phenothiazine. Melting point 127-129 °C.

Example IV: N-(4-Tert-butylphenylthiomethylene)phenothiazine

The compound of Example IV is prepared by following the general procedure of Example I and reacting 4-tert-butylthiophenyl with formaldehyde and phenothiazine.

Example V: N-(2,4-Dimethylphenylthiomethylene)phenothiazine

The compound of Example V is prepared by following the general procedure of Example I and reacting 2,4-dimethylthiophenol with formaldehyde and phenothiazine.

Example VI: N-(4-Methoxyphenylthiomethylene)phenothiazine

The compound of Example VI is prepared by following the general procedure of Example I and reacting 4-methoxythiophenol with formaldehyde and phenothiazine.

Example VII: N-(2-Naphthylthiomethylene)phenothiazine

The compound of Example VII is prepared by following the general procedure of Example I and reacting 2-naphthylmercaptan with formaldehyde and phenothiazine.

Example VIII: Engine Oil Thin Film Oxygen Uptake Test

This test is conducted in a standard rotary bomb apparatus (described in ASTM D-2272) with modifications in procedure as described in the Preprint No. 82 CC-10-1, presented at the Conference of the American Society of Lubrication Engineers, October 5-7, 1982.

A 1.5 ml test sample of 150 N paraffinic mineral oil containing enough zinc dialkyldithiophosphate (ZDTP) to give 0.1 % by weight of zinc and 0.5 % by weight of the test compound is placed in the test apparatus. To the above is added a catalyst package comprising 0.075 grams of oxidized fuel components, 0.075 grams of soluble metal catalyst* and 0.030 grams of water. The temperature is set at 160 °C and the initial oxygen pressure is 90 psi (620 kPa). Failure is taken as the time in minutes for a pressure drop of 25 psi (172 kPa) to be observed. The testresults are given below.

| Test Compound of Example | Failure Time (minutes) |
|---|---|
| Base oil | 99-105 |
| I | 138 |
| II | 145 |
| III | 186 |

*The soluble metal catralysts are a mixture of the following metal naphthenates in the weight ratios given below: cupric 0.69 %, ferric 0.41 %, lead 8.0 %, manganese 0.35 %, stannous 0.36 % (as naphthenates).

The data thus show a significant improvement in stability toward oxidation when the instant compounds are added to oil.

In summary, this invention provides a novel class of lubricant antioxidants which exhibit excellent antioxidative performance. Variations may be made in procedures, proportions and materials without departing from the scope of the invention as defined in the following claims.

**Claims**

1. A lubricant composition comprising a mineral oil or a synthetic oil or mixtures thereof and an effective stabilizing amount of a compound of the formula

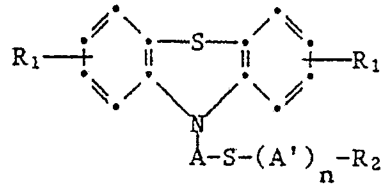

wherein $R_1$ are independently hydrogen, alkyl of 1 to 24 carbon atoms, cycloalkyl of 5 to 7 carbon atoms or aralkyl of 7 to 9 carbon atoms;

A and A' are independently alkylene of 1 to 8 carbon atoms;
n is 0 or 1; and
$R_2$ is alkyl of 1 to 18 carbon atoms, cycloalkyl of 5 to 7 carbon atoms, said cycloalkyl substituted by alkyl of 1 to 18 carbon atoms, phenyl, phenyl substituted by alkyl, alkoxy or thioalkyl to a total carbon atom content of 7 to 30, naphthyl or naphthyl substituted by alkyl, alkoxy or thioalkyl to a total carbon atom content of 11 to 40, with the proviso that A is not ethylene when n is zero and $R_2$ is alkyl of 6 to 12 carbon atoms or phenyl.

2. The lubricant composition of claim 1, wherein $R_1$ are independently hydrogen or alkyl of 1 to 12 carbon atoms.

3. The lubricant composition of claim 1, wherein A and A' are independently

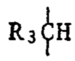

wherein $R_3$ is alkyl of 1 to 7 carbon atoms.

4. The lubricant composition of claim 1, wherein $R_2$ is alkyl of 4 to 12 carbon atoms, phenyl or phenyl mono-, di- or trisubstituted by alkyl of 1 to 8 carbon atoms or one of said substituents being alkoxy or thioalkyl of 1 to 12 carbon atoms.

5. The lubricant composition of claim 1, wherein said compound is N-(phenylthiomethylene)phenothiazine.

6. The lubricant composition of claim 1, wherein said compound is N-(n-octylthiomethylene)phenothiazine.

7. A compound of the formula

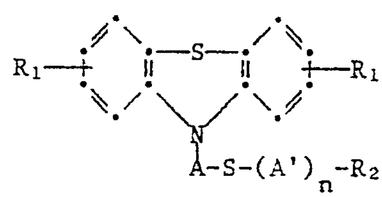

wherein $R_1$ are independently hydrogen, alkyl of 1 to 24 carbon atoms, cycloalkyl of 5 to 7 carbon atoms or aralkyl of 7 to 9 carbon atoms;

A and A' are independently alkylene of 1 to 8 carbon atoms;
n is 0 or 1; and
$R_2$ is cycloalkyl of 5 to 7 carbon atoms, said cycloalkyl substituted by alkyl of 1 to 18 carbon atoms, phenyl, phenyl substituted by alkyl, alkoxy or thioalkyl to a total carbon atom content of 7 to 30, naphthyl or naphthyl substituted by alkyl, alkoxy or thioalkyl to a total carbon atom content of 11 to 40, with the proviso that A is not ethylene when n is zero and $R_2$ is alkyl of 6 to 12 carbon atoms or phenyl.

8. The compound of claim 7, wherein $R_1$ are independently hydrogen or alkyl of 1 to 12 carbon atoms.

9. The compound of claim 7, wherein A and A' are independently

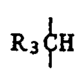

wherein $R_3$ is alkyl of 1 to 7 carbon atoms.

**10.** The compound of claim 7, wherein $R_2$ is phenyl or phenyl mono-, di- or trisubstituted by alkyl of 1 to 8 carbon atoms or one of said substituents being alkoxy or thioalkyl of 1 to 12 carbon atoms.

**11.** Use of compounds according to the formula of claim 1 as stabilizer against oxidative degeneration in lubricant compositions.

**Revendications**

**1.** Composition lubrifiante comprenant une huile minérale ou une huile synthétique ou un mélange de celles-ci, et une quantité à effet stabilisant d'un composé de la formule

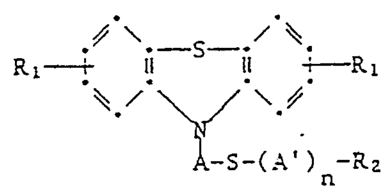

dans laquelle

les symboles $R_1$ représentent chacun indépendamment l'hydrogène, un groupe alkyle de 1 à 24 atomes de carbone, cycloalkyle de 5 à 7 atomes de carbone ou aralkyle de 7 à 9 atomes de carbone ;

A et A' représentent chacun indépendamment un groupe alkylène de 1 à 8 atomes de carbone ;

n est 0 ou 1 ; et

$R_2$ est un groupe alkyle de 1 à 18 atomes de carbone, cycloalkyle de 5 à 7 atomes de carbone, ledit groupe cycloalkyle substitué par un groupe alkyle de 1 à 18 atomes de carbone, phényle, phényle substitué par un ou plusieurs groupes alkyle, alcoxy ou thioalkyle jusqu'à un nombre total d'atomes de carbone de 7 à 30, naphtyle ou naphtyle substitué par un ou plusieurs groupes alkyle, alcoxy ou thioalkyle jusqu'à un nombre total d'atomes de carbone de 11 à 40, à condition que A ne soit pas un groupe éthylène lorsque n est zéro et $R_2$ est un groupe alkyle de 6 à 12 atomes de carbone ou phényle.

**2.** Composition lubrifiante selon la revendication 1, dans laquelle les symboles $R_1$ représentent chacun indépendamment l'hydrogène ou un groupe alkyle de 1 à 12 atomes de carbone.

**3.** Composition lubrifiante selon la revendication 1, dans laquelle A et A' représentent chacun indépendamment

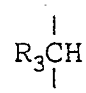

où $R_3$ est un groupe alkyle de 1 à 7 atomes de carbone.

**4.** Composition lubrifiante selon la revendication 1, dans laquelle $R_2$ est un groupe alkyle de 4 à 12 atomes de carbone, phényle ou phényle mono-, di- ou trisubstitué par des groupes alkyle de 1 à 8 atomes de carbone ou dont l'un desdits substituants est un groupe alcoxy ou thioalkyle de 1 à 12 atomes de carbone.

**5.** Composition lubrifiante selon la revendication 1, dans laquelle ledit composé est la N-(phénylthiométhylène)phénothiazine.

**6.** Composition lubrifiante selon la revendication 1, dans laquelle ledit composé est la N-(n-octylthiométhylène)phénothiazine.

**7.** Composé de la formule

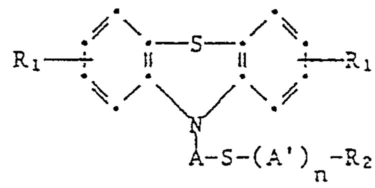

dans laquelle

les symboles $R_1$ représentent chacun indépendamment l'hydrogène, un groupe alkyle de 1 à 24 atomes de carbone, cycloalkyle de 5 à 7 atomes de carbone ou aralkyle de 7 à 9 atomes de carbone ;

A et A' représentent chacun indépendamment un groupe alkylène de 1 à 8 atomes de carbone ;

n est 0 ou 1 ; et

$R_2$ est un groupe cycloalkyle de 5 à 7 atomes de carbone, ledit groupe cycloalkyle substitué par un groupe alkyle de 1 à 18 atomes de carbone, phényle, pnényle substitué par un ou plusieurs groupes alkyle, alcoxy ou thioalkyle jusqu'à un nombre total d'atomes de carbone de 7 à 30, naphtyle ou naphtyle substitué par un ou plusieurs groupes alkyle, alcoxy ou thioalkyle jusqu'à un nombre total d'atomes de carbone de 11 à 40,

à condition que A ne soit pas un groupe éthylène lorsque n est zéro et $R_2$ est un groupe alkyle de 6 à 12 atomes de carbone ou phényle.

8. Composé selon la revendication 7, dans lequel les symboles $R_1$ représentent chacun indépendamment l'hydrogène ou un groupe alkyle de 1 à 12 atomes de carbone.

9. Composé selon la revendication 7, dans lequel A et A' représentent chacun indépendamment

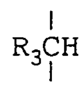

où $R_3$ est un groupe alkyle de 1 à 7 atomes de carbone.

10. Composé selon la revendication 7, dans lequel $R_2$ est un groupe phényle ou phényle mono-, di- ou trisubstitué par des groupes alkyle de 1 à 8 atomes de carbone ou dont l'un desdits substituants est un groupe alcoxy ou thioalkyle de 1 à 12 atomes de carbone.

11. Utilisation de composés répondant à la formule de la revendication 1 comme stabilisants contre la dégradation par oxydation dans des compositions lubrifiantes.

## Patentansprüche

1. Schmierstoffzusammensetzung enthaltend ein Mineralöl oder ein synthetisches Oel oder Mischungen davon und eine wirksame stabilisierende Menge einer Verbindung der Formel

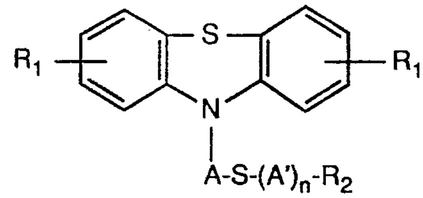

worin die $R_1$ unabhängig voneinander Wasserstoff, Alkyl mit 1 bis 24 C-Atomen, Cycloalkyl mit 5 bis 7 C-Atomen oder Aralkyl mit 7 bis 9 C-Atomen bedeuten;

A und A' unabhängig voneinander Alkylen mit 1 bis 8 C-Atomen darstellen,

n 0 oder 1 ist, und

$R_2$ Alkyl mit 1 bis 18 C-Atomen, Cycloalkyl mit 5 bis 7 C-Atomen, mit Alkyl mit 1 bis 18 C-Atomen substituiertes Cycloalkyl mit 5 bis 7 C-Atomen, Phenyl, mit Alkyl, Alkoxy oder Thioalkyl bis zu einer

Gesamt-C-Atomzahl von 7 bis 30 substituiertes Phenyl, Naphthyl oder mit Alkyl, Alkoxy oder Thioalkyl bis zu einer Gesamt-C-Atomzahl von 11 bis 40 substituiertes Naphthyl bedeutet, mit der Massgabe, dass A nicht für Ethylen steht, wenn n Null und $R_2$ Alkyl mit 6 bis 12 C-Atomen oder Phenyl bedeuten.

2. Schmierstoffzusammensetzung nach Anspruch 1, worin die $R_1$ unabhängig voneinander Wasserstoff oder Alkyl mit 1 bis 12 C-Atomen bedeuten.

3. Schmierstoffzusammensetzung nach Anspruch 1, worin A und A' unabhängig voneinander

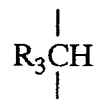

bedeuten, worin $R_3$ Alkyl mit 1 bis 7 C-Atomen ist.

4. Schmierstoffzusammensetzung nach Anspruch 1, worin $R_2$ Alkyl mit 4 bis 12 C-Atomen, Phenyl oder durch Alkyl mit 1 bis 8 C-Atomen mono-, di- oder trisubstituiertes Phenyl bedeutet, wobei einer dieser Substituenten auch Alkoxy oder Thioalkyl mit 1 bis 12 C-Atomen sein kann.

5. Schmierstoffzusammensetzung nach Anspruch 1, worin die genannte Verbindung N-(Phenylthiomethylen)phenothiazin ist.

6. Schmierstoffzusammensetzung nach Anspruch 1, worin die genannte Verbindung N-(n-Octylthiomethylen)phenothiazin ist.

7. Verbindungen der Formel

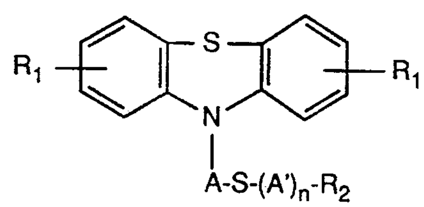

worin die $R_1$ unabhängig voneinander Wasserstoff, Alkyl mit 1 bis 24 C-Atomen, Cycloalkyl mit 5 bis 7 C-Atomen oder Aralkyl mit 7 bis 9 C-Atomen;
A und A' unabhängig voneinander Alkylen mit 1 bis 8 C-Atomen;
n 0 oder 1; und
$R_2$ Cycloalkyl mit 5 bis 7 C-Atomen, mit Alkyl mit 1 bis 18 C-Atomen substituiertes Cycloalkyl mit 5 bis 7 C-Atomen, Phenyl, mit Alkyl, Alkoxy oder Thioalkyl bis zu einer Gesamt-C-Atomzahl von 7 bis 30 substituiertes Phenyl, Naphthyl oder mit Alkyl, Alkoxy oder Thioalkyl bis zu einer Gesamt-C-Atomzahl von 11 bis 40 substituiertes Naphthyl bedeuten, mit der Massgabe, dass A nicht für Ethylen steht, wenn n Null und $R_2$ Alkyl mit 6 bis 12 C-Atomen oder Phenyl bedeuten.

8. Verbindungen nach Anspruch 7, worin die $R_1$ unabhängig voneinander Wasserstoff oder Alkyl mit 1 bis 12 C-Atomen bedeuten.

9. Verbindungen nach Anspruch 7, worin A und A' unabhängig voneinander

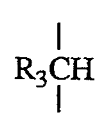

bedeuten, worin $R_3$ für Alkyl mit 1 bis 7 C-Atomen steht.

**10.** Verbindungen nach Anspruch 7, worin $R_2$ Phenyl oder durch Alkyl mit 1 bis 8 C-Atomen mono-, di- oder trisubstituiertes Phenyl bedeutet, wobei einer dieser Substituenten auch Alkoxy oder Thioalkyl mit 1 bis 12 C-Atomen sein kann.

**11.** Verwendung von Verbindungen der in Anspruch 1 angegebenen Formel als Stabilisatoren gegen oxidativen Abbau in Schmierstoffzusammensetzungen.